# EUROPEAN PATENT APPLICATION

(11) **EP 1 566 185 A1**
(43) Date of publication of application: **24.08.2005**
(21) Application number: 03758758.1
(22) Date of filing: 22.10.2003
(51) Int. Cl.: A61L 2/26

(54) **STORAGE DEVICE FOR STEAM STERILIZATION**

(30) Priority: 26.11.2002 JP 2002342621
(71) Applicant: Olympus Corporation, Tokyo 151-0072 (JP)
(72) Inventor: KUROSHIMA, Hisashi, Hachioji-shi, Tokyo 193-0832 (JP); SUZUKI, Eiri, Sagamihara-shi, Kanagawa 229-0038 (JP); HASEGAWA, Hitoshi, Yokohama-shi, Kanagawa 222-0002 (JP); NOGUCHI, Toshiaki, Tachikawa-shi, Tokyo 190-0002 (JP)
(74) Representative: von Hellfeld, Axel
(86) International application number: PCT/JP2003/013464
(87) International publication number: WO 2004/047876

(57) **Abstract**

A steam sterilization storing device comprises a cooling holding unit for holding and cooling sterilized equipment which has been sterilized by hot steam such as endoscopes, and at least one of a drying holding unit for holding and drying the sterilized equipment and a storage unit for holding and storing the sterilized equipment.

## Description

### Technical Field

The present invention relates to a steam sterilization storing device which efficiently cools equipment that has been sterilized with hot steam.

### Background Art

In recent years, sterilizing medical equipment such as endoscopes and the like with high-temperature and high-pressure steam, which is low in running costs, is becoming the primary method for such sterilization. For example, Japanese Unexamined Patent Application Publication No. 5-285103 discloses a high-temperature high-pressure steam sterilization device, i.e., a so-called autoclave, for sterilizing endoscopes (also abbreviated to "scope") with high-temperature and high-pressure steam.

With a scope subjected to high-temperature and high-pressure steam sterilization, and a container storing such a scope, the temperature thereof becomes high, and accordingly, there is the need to efficiently cool the scope and the container for containing such a scope in order to improve running efficiency so as to be able to use the scope and the container in endoscopy at a short time.

However, arrangements according to conventional techniques have the disadvantage of taking a long time to restore the state of the scope and so forth to be usable again.

It is an object of the present invention to provide a steam sterilization storing device and a steam sterilization system suitable for performing processing such as cooling processing or the like in order to restore the state of a device such as a scope or the like which is to be subjected to steam sterilization, so that the device can be used again.

### Disclosure of Invention

A steam sterilization storing device according to the present invention comprises: cooling holding means for holding and cooling sterilized equipment which has been sterilized by hot steam; and at least one of drying holding means for holding and drying the sterilized equipment and storage means for holding and storing the sterilized equipment.

A steam sterilization system according to the present invention comprises: a high-temperature high-pressure steam sterilization device for storing and sterilizing equipment to be sterilized with hot steam; a transporting device for holding and transporting the sterilized equipment sterilized in the high-temperature high-pressure steam sterilization device; and a cooling holding device for holding and cooling sterilized equipment which has been sterilized by the high-temperature high-pressure steam sterilization device.

### Brief Description of the Drawings

Figs. 1A through 2 relate to a first embodiment of the present invention, wherein Fig. 1A is a schematic overall configuration drawing of a storage unit capable of cooling, drying, and storing;
Fig. 1B is a plan view of a placement example of a scope in a cooling holding bin;
Fig. 1C is a perspective view illustrating the scope stored in a tray;
Fig. 1D is a perspective view illustrating lowered portions provided in a grating table;
Fig. 2 is a perspective view illustrating a portion of the storage unit;
Fig. 3 is a perspective view illustrating the configuration of the storage unit having cooling means according to a modification;
Figs. 4 through 7 relate to a second embodiment of the present invention, wherein Fig. 4 is a perspective view illustrating a steam sterilization system having a storage rack unit with cooling functions, according to the second embodiment of the present invention;
Fig. 5 is a perspective view illustrating a tray transporting device holding the tray storing the scope;
Fig. 6 is a perspective view illustrating the tray transporting device set to a storage state; and
Fig. 7 is a perspective cutaway view illustrating the configuration of the storage rack unit with cooling functions.

### Best Mode for Carrying Out the Invention

The following is a description of embodiments of the present invention, with reference to the drawings. A storage unit according to a first embodiment of the steam sterilization device according to the present invention will be described with reference to Figs. 1A through 2.

The object of the present invention is to provide a storage unit capable of cooling, drying, and storing medical equipment to be sterilized by steam under high temperature and high pressure, such as endoscopes (hereafter referred to simply as "scopes") and the like. The following configuration enables reprocessing, which facilitates effective reuse of scopes and the like.

As shown in Fig. 1A, a storage unit 33 comprises cooling holding bins 34 which cool as well as hold scopes and the like, a drying holding bin 35 which dries the scopes and the like, and storage bins 36 which store the scopes and the like. The cooling holding bins 34, drying holding bin 35, and storage bins 36 are formed in a single main unit 32, arrayed in the vertical direction, for example. With this arrangements, the cooling holding bins 34 and drying holding bin 35 are partitioned vertically, but the air channels are shared as described later, to reduce size. Also, the drying holding bin 35 and storage bins 36 are completely partitioned vertically.

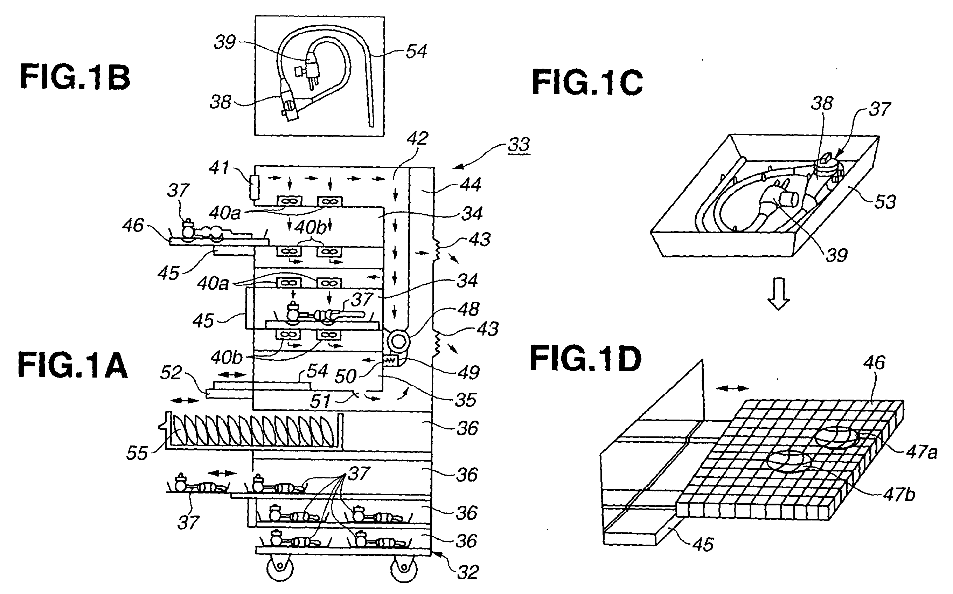

The cooling holding bins 34 comprise small fans 40a and 40b provided in the ceiling face and bottom face thereof, positioned so as to be above and below a scope operating unit 38 and a connector unit 39 as shown in Fig. 1B upon a sterilized scope 37 being stored therein as equipment to be sterilized, for example. The small fans 40a installed in the ceiling faces are connected to a channel 42 communicating with a dust-removing filter 41, and the small fans 40a operate so as to blow air down upon the scope 37 positioned below. The small fans 40b installed in the bottom faces are connected to a channel 44 communicating with a vent duct 43 of the storage unit 33, and the small fans 40b operate so as to suck air away from area where the scope 37 is situated. Accordingly, the air flows from the ceilings of the cooling holding bins 34 toward the bottom faces thereof, so the air blowing on the scope 37 is clean air with no foreign matter.

The cooling holding bins 34 have mesh or grid-like grating tables 46 which can be pulled out upon opening a door 45, as shown in Fig. 1D, with lowered portions 47a and 47b formed at the positions of the grating tables 46 between the small fans 40a and 40b situated facing one another from above and below the grid-like grating tables 46. The lowered portions 47a and 47b both dip downwards. The grating tables 46 are not restricted to a mesh form, rather any form may be used so long as air readily passes through.

Also, trays 53 to be placed on the grating tables 46 are arranged so that the overall manner in which the scope 37 is to be stored is predetermined, as shown in Fig. 1C. Specifically, the tray 53 has multiple protruding pins along the outer shape of the scope 37 when placed therein, so the scope 37 is positioned in the tray 53 by being placed between the pins. In this case, the positions of the scope operating unit 38 and the connector unit 39 are set to be the same for scopes 37 with different lengths of the inserting portions and so forth.

Upon the user placing the tray 53, in which the scope 37 has been generally positioned and held, onto the grating table 46, the positions of the scope operating unit 38 and the connector unit 39 are at the lowered portions 47a and 47b as shown in Fig. 1D. The user then stores the grating table 46 within the cooling holding bin 34, so as to be placed between the small fans 40a and 40b, and effectively cooled.

The drying holding bin 35 is provided with: an opening 50 communicating with the channel 42 which communicates with the small fan 40a set on the ceiling side of the cooling holding bin 34 via a sirocco fan 48 and a heater 49; and an opening 51 connected to the channel 44 communicating with the small fan 40b provided on the bottom face of the cooling holding bin 34. Racks 53 which can be pulled out when a door 52 is opened are provided within the drying holding bin 35.

The storage bins 36 are a drawer area for storing the scopes 37 following being cooled in the cooling holding bin 34, arranged so as to readily store the scopes 37. An equipment 55, such as treatment equipment other than the scopes 37, and cleaning tools and the like, may also be stored therein.

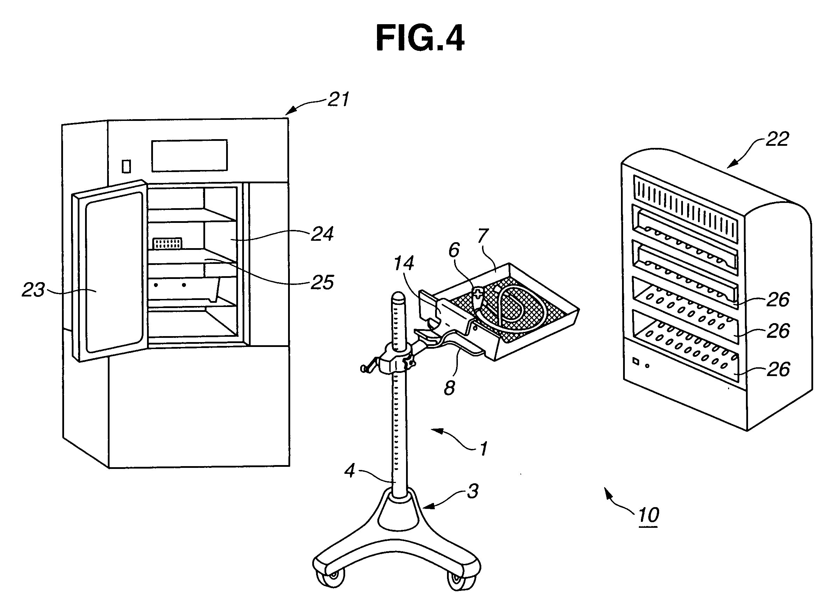

Next, an example of using the above-described storage unit 33 will be described. The scope 37 used for examination of a patient is cleaned by manual washing or by a washing device. Subsequently, the scope 37 is placed in a high-temperature high-pressure steam sterilization device 21 shown in Fig. 4, for example, for the purpose of sterilization. Now at the time of placing the scope 37 in the high-temperature high-pressure steam sterilization device 21, in the event that the scope 37 is wet, the sterilizing functions of the high-temperature high-pressure steam sterilization device 21 may be lowered, thereby requiring a long sterilization period to compensate for this. To avoid this, the channels and the outer face of the scope 37 must be sufficiently dried.

Now, the user can place the scope 37 which is to be sterilized in the drying holding bin 35 of the storage unit 33 according to the present embodiment, thereby drying the scope 37 in a relatively short time with the warm air blowing out from the opening 50 within the drying holding bin 35.

The areas of the openings 50 and 51 are sufficiently large, and dry and hot air always enters the drying holding bin 35 and passes out from the opening 51 to the vent duct 43, so scopes 37 can be dried efficiently. Dried scopes 37 are placed in the tray 53 and either peeled back or placed in the high-temperature high-pressure steam sterilization device 21 as they are.

The scopes 37 subjected to high-temperature high-pressure sterilization are removed from the device, and placed in the storage unit 33 again. The door 45 of the cooling holding bin 34 is opened, the grating table 46 is pulled out, and the scope 37 is placed thereupon.

An arrangement wherein the operating unit 38 and connector unit 39 of the scope 37 are situated in the lowered portions 47a and 47b of the grating table 46 allows the operating unit 38 and connector unit 39 to come between the small fans 40a and 40b disposed in the ceiling face and bottom face in a reliable manner when the grating table 46 is stored in the holding bin 34.

The reason that the operating unit 38 and connector unit 39 are placed between the small fans 40a and 40b is to improve the cooling efficiency of the scope 37 by intensively cooling these portions. There is heat in the space within the scope 37 to be cooled, so aggressively cooling the operating unit 38 and connector unit 39 which are formed of resin and metal which are not readily cooled due to the large space therein improves the cooling efficiency of the entire scope 37.

On the other hand, an inserting portion 54 shown in Fig. 1B is relatively quickly cooled even if let standing, and accordingly there is no need to actively cool this part.

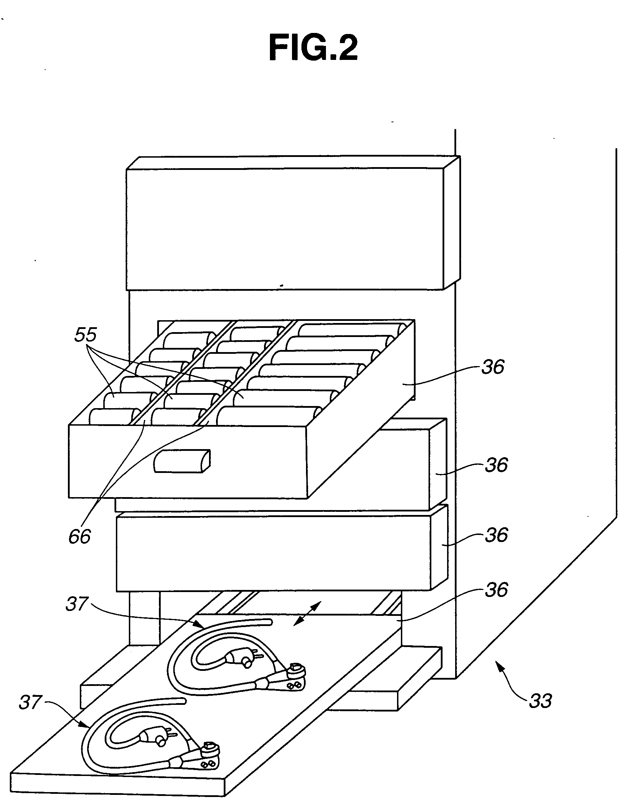

As shown in Fig. 2, the equipment 55 other than scopes 37 such as treatment equipment or cleaning tools and the like when stored in the storage unit 33 can also be effectively air-cooled by placing in the lowered portions 47a and 47b.

Further, in the event of storing or holding the equipment 55 other than the scopes 37, such as treatment equipment or cleaning tools and the like, in the storage bins 36 as shown in Fig. 2, the storage bins 36 may be sectioned with sectioning members 66 so as to form areas corresponding to the size of the equipment 55 to be stored, thereby enabling the equipment 55 to be efficiently held.

The scope 37 stored in the cooling holding bin 34 may be cooled by the actions of the small fans 40a and 40b, the heater 49, and the sirocco fan 48, upon operating a front panel (not shown) on the storage unit 33.

The scope 37 will be cool enough for the user to handle in a little more than five minutes after starting the cooling process if the scope 37 is in a stripped-down state, or a little more than twenty minutes if in a peeled-back state, so a timer starts running after the fan 48 is operated and a buzzer sounds after a set amount of time, to notify the user of this. The timer may also be set according to the work flow of the user.

The cooled scope 37 has thus been washed, sterilized, cooled, and so forth, thereby ending reprocessing, so the scope 37 is ready to be used again. In the event that the scope 37 is to be used for endoscope examination or the like immediately, the scope 37 is carried to the endoscope examination room, otherwise, the scope 37 is placed in the area for storing scopes 37 in the storage bins 36. Also storing equipment 55 such as treatment equipment or cleaning tools and the like in the storage bins 36 along with the scopes 37 facilitates reuse thereof, since these instruments and the like are also an integral part of the flow of repeated endoscope examination and reprocessing.

According to the present embodiment, the single main unit 32 comprises the cooling holding bins 34, the drying holding bin 35, and the storage bins 36, so the drying and cooling steps necessary in the process of washing and hot-steam sterilizing equipment to be sterilized for reuse such as the scopes 37 can be performed very easily, without requiring the instruments being sterilized to be carried around. The equipment becomes reusable in a shorter time, and further, can be stored in the unit if not to be used for endoscope examination immediately.

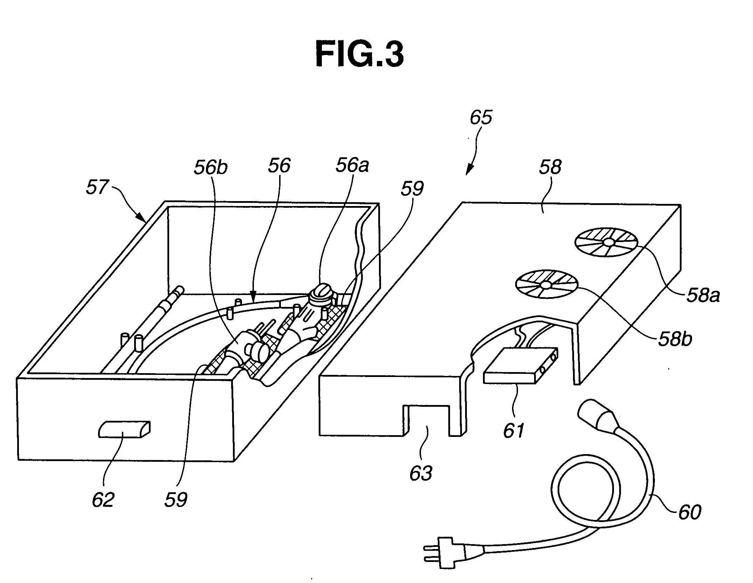

While the above description has been made with regard to a case wherein the scopes 37 stored in the trays 53 and subjected to high-temperature steam sterilization are held in the cooling holding bins 34 where they are cooled, an arrangement may be made wherein a lidded holding unit 65 having cooling means is held in the storage bins 36, as shown in Fig. 3. This lidded holding unit 65 will now be described with reference to Fig. 3.

Fig. 3 illustrates the lidded holding unit 65 which holds the scope sterilized by the high-temperature high-pressure steam sterilization device, and which has cooling means. The lidded holding unit 65 achieves the object of efficiently cooling the scope sterilized with hot steam.

The lidded holding unit 65 shown in Fig. 3 is used for performing sterilization with the high-temperature high-pressure steam sterilization device, and comprises a tray 57 which opens at the top for storing a scope 56, and a lid 58 which can be closed so as to cover the top. The lid 58 has small fans 58a and 58b at positions capable of blowing air directly down onto the operating unit 56a and connector unit 56b of a scope 56 held in the tray 57.

Also, the positions on the bottom of the tray 57 where the operating unit 56a and connector unit 56b of the scope 56 are to be situated are a mesh 59, so as to pass through the breeze from the fans 58a and 58b directly above.

An electric power cable 60 for supplying electric power to the small fans 58a and 58b can be connected to the lid 58. A battery 61 is built into the lid 58 to increase the freedom of use, so that the lid 58 can be moved and still used, with an unshown charger recharging the battery 61.

Also, a protrusion 62 is provided on the tray 57 side and a notch 53 is provided on the lid 58 side to restrict the attachment position, so that the small fans 58a and 58b always come directly above the operating unit 56a and connector unit 56b when the lid 58 is used to cover the tray 57. For example, the tray 57 and lid 58 are rectangular shapes when viewed in planar fashion, and are attached so as to fit one to another. Providing the protrusion 62 on the tray 57 side and the notch 53 on the lid 58 side to restrict the attachment position ensures that the lid will not be put on backwards. Of course, the notch may be provided on the tray 57 side and the protrusion on the lid 58 side, instead.

Next, the operations of the holding unit 65 will be described along with a usage example thereof.

The scope 56 subjected to high-temperature high-pressure sterilization in the tray 57 is extracted from the high-temperature high-pressure steam sterilization device 21 shown in Fig. 4 (described later) by a tray transportation device 1. Subsequently, the lid 58 is placed on the tray 57, and the small fans 58a and 58b are started by pressing an unshown switch provided on the lid 58.

Thus, the operating unit 56a and connector unit 56b of the scope 56 are intensively cooled, resulting in the scope 56 being efficiently cooled. An unshown timer is provided to the holding unit 65, to operate such that the small fans 58a and 58b stop when a predetermined time elapses, so the holding unit 65 can be placed in a storage unit in an endoscope reprocessing room or in the storage bins 36 of the storage unit 33, to be cooled for a predetermined time and then stored, with no troublesome relocating. The scope has been cooled to body temperature or room temperature at the end of the time set by the timer, and accordingly can be immediately reused for endoscope examination, in a short time.

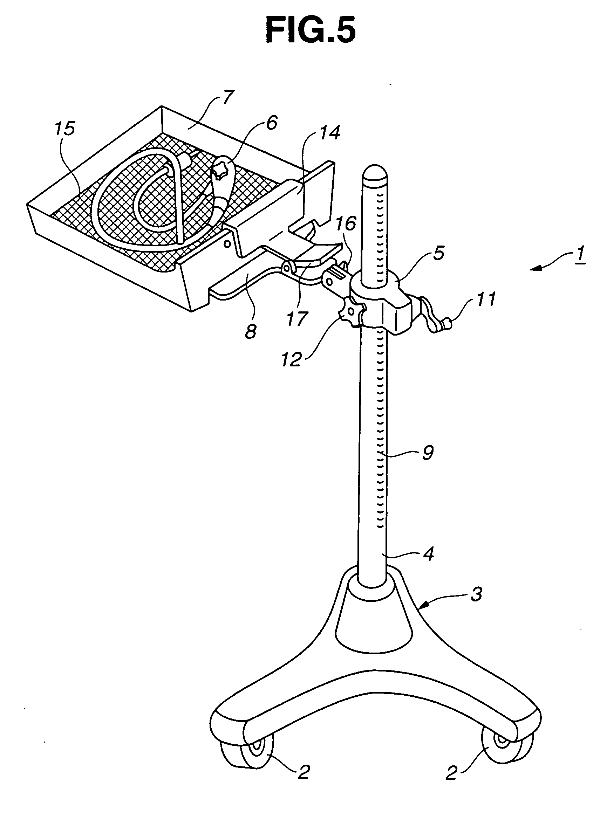

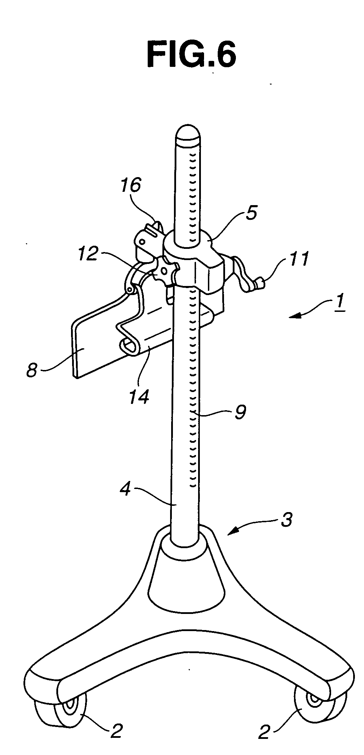

Next, the tray transporting device for steam sterilizing will be described with reference to Figs. 5 and 6. This tray transporting device realizes an arrangement wherein a container storing sterilized equipment such as scopes and the like, sterilized with hot steam, can be extracted without being touched by the user.

As shown in Fig. 5, the tray transporting device 1 has a transporting device base (hereafter referred to simply as "base") 3 with wheels 2 provided on the bottom thereof so as to be movable. A stay member (shaft) 4 is erected from the base 3. A slide unit 5 capable of sliding in the longitudinal direction of the stay member 4 is provided thereto, the slide unit 5 having a hanger member 8 for supporting the bottom of a tray 7 holding sterilized equipment such as a scope 6 or the like sterilized with hot steam. A clamp mechanism 14 is also formed to cooperate with the hanger member 8 so as to clamp or unclamp the tray 7. The tray 7 storing the scope 6 or the like is to be placed in the high-temperature high-pressure steam sterilization device 21, and accordingly is constructed so as to endure high-temperature high-pressure sterilization.

The bottom portion of the base 3 extends in three directions horizontally, for example, and wheels 2 for following over the floor or the like are attached to each. The rod-shaped stay member 4 is erected at the center of the base, and guide marks 9 are engraved or stamped on the stay member 4 so that the slide unit 5 can be moved along the stay member 4 in the longitudinal direction by a handle 11 for adjusting the height. The slide unit 5 has a hole through which the stay member 4 is passed, and can be completely removed from the stay member 4. The handle 11 is attached to the slide unit 5.

The user rotates the handle 11, whereby an unshown gear within the slide unit 5 which engages with the guide marks 9 on the stay member 4 is rotated, thereby moving the slide unit 5 in the longitudinal direction of the stay member 4, i.e., in the vertical direction. This forms a height adjusting mechanism whereby the height of the slide unit 5 can be adjusted. Once a desired height is reached, the slide unit 5 is fixed to the stay member 4 by rotating a fixing screw 12.

The slide unit 5 has the hanger member 8 for supporting the tray 7 in which the scope 6 is stored. The hanger member 8 is provided with the clamp mechanism 14 for clamping or unclamping the tray 7. The tray 7 storing the scope 6 is placed in the high-temperature high-pressure steam sterilization device 21 (see Fig.4). Therefore, the tray 7 is provided with the endurance for high-temperature high-pressure steam sterilization.

The tray 7 has the form of a shallow box with the top opened, and a bottom face 15 thereof where the scope 6 or the like is to be placed is formed of a mesh so as to reduce the amount of contact with this face and also to maximize passage of air, so that the effects of high-temperature high-pressure sterilization are optimal. The tray 7 is completely formed of stainless steel or heat-resistant resin or the like, so as to sufficiently endure the high-temperature high-pressure steam, and also resist rust under the steam environment.

The clamp mechanism 14 for clamping and unclamping the tray 7 comprises a clamping member which cooperates with the hanger member 8 at a fulcrum portion formed toward the base side thereof so as to open and close. The hanger member 8 supports the bottom 15 of the tray 7, and the clamp member has a U-shaped or like shape capable of engaging a side of the tray from above, and is pressed by an unshown spring in the direction of closing against the tray 7.

The tip of the clamp member opens as to the hanger member 8 by the user pressing a portion behind the fulcrum portion, such as an opening/closing operating portion 17 formed at the rear end of the clamp member for example, and this can be used to clamp or unclamp the tray 7.

The hanger member 8 and the clamp mechanism 14 may be formed of stainless steel, ceramics having high heat endurance and low heat conductivity, another material coated with a ceramic film, or the like. In any case, at least the opening/closing operating portion 17 and other portions at the rear end of the clamp member should be formed of a material which does not readily conduct heat.

The hanger member 8 can be fixed to the slide unit 5 in the direction of being erected at right angles to the stay member 4. In the event of holding the tray 7, the hanger member 8 is erected in advance. Namely, the hanger member 8 is fixed to the slide unit 5 so as to be perpendicular to the stay member 4 extended in upward and downward directions, i.e., so as to be horizontally erected.

Therefore, the base end of the hanger member 8 is fixed to the slide unit 5 by a fixing screw 16 to fix the hanger member 8 side. In further detail, a plate-shaped tab is formed on the base of the hanger member 8 and the tab is inserted into a slot provided to the slide unit 5 so as to be rotatable on the fixing screw 16, and tightening the fixing screw 16 fixes the hanger member 8.

In the event of storing the tray transporting device 1, in a case of being hard to store in a compact manner if the hanger member 8 is kept upright, the fixing screw 16 may be loosened so that the hanger member 8 droops down as shown in Fig. 6 so as to be generally parallel with the stay member 4, thereby saving storage space.

In this way, the tray transporting device 1 has a hanger member 8 with the clamp mechanism 14 for clamping and unclamping trays 7 connected to the slide unit 5 attached to the stay member 4 of the base 3 to which wheels 2 are provided, and accordingly, a transporting mechanism is formed whereby hot trays 7 can be clamped and removed from the high-temperature high-pressure steam sterilization device 21, and transported to a desired location.

This is a great improvement over conventional configurations wherein the user had to wear thermal gloves to remove hot scopes or containers containing the scopes, sterilized in a high-temperature high-pressure steam sterilization device. This tray transporting device 1 solves the conventional problem in a way which does not necessitate troublesome procedures.

Next, a steam sterilization system 10 comprising the tray transporting device 1, the high-temperature high-pressure steam sterilization device 21, and a holding rack unit 22 having cooling functions, will be described with reference to Fig. 4.

The high-temperature high-pressure steam sterilization device 21 of the steam sterilization system 10 has a high-temperature high-pressure steam sterilization chamber (hereafter simply referred to as "steam sterilization chamber") 24 within a door 23, with metal racks 25 or the like for loading trays 7 wherein the scopes 6 are stored.

The user places trays 7 wherein the scopes 6 to be sterilized are stored on the racks 25 in the steam sterilization chamber 24, closes the door 23, and turns the electric power switch on. The steam sterilization chamber 24 is filled with high-temperature high-pressure steam and so forth to sterilize the scopes 6 along with the trays 7 with the high-temperature high-pressure steam.

As for conditions for effecting high-temperature high-pressure steam sterilization, hot steam of around 121 to 135°C is brought into contact with the equipment to be sterilized. For example, American National Standards Institute AAMIST 37-1992 stipulates 4 minutes at 132°C under pressure of approximately 2 atm. for a sterilization process. This sterilization process subjects germs and the like on the equipment to be sterilized to the high-temperature high-pressure steam, effecting sterilization. Accordingly, the surface of the scope 6 immediately following the high-temperature high-pressure steam sterilization is hot (e.g., 90 to 100°C).

The tray transporting device 1 allows the trays 7 holding the scopes 6 immediately following the high-temperature high-pressure steam sterilization to be removed without the user having to use thermal gloves or the like, and further, the trays 7 holding the scopes 6 can be stored in the holding rack unit 22 having cooling functions, so that the user can use the scopes 6 again in a short time.

After high-temperature high-pressure steam sterilization in the high-temperature high-pressure steam sterilization device 21 is completed, the user opens the door 23 as shown in Fig. 4, brings the hanger member 8 of the tray transporting device 1 up to the tray 7 loaded on the rack 25 in the steam sterilization chamber 24 within the high-temperature high-pressure steam sterilization device 21, opens the tip of the clamp mechanism 14, and securely grasps the tray 7 with the hanger member 8. As described above, the clamp mechanism 14 is operated by pressing or releasing the opening/closing operating portion 17 at the rear end of the clamp mechanism 14. Thus, the user can grasp the tray 7 with the tip of the clamp mechanism 14, without having to directly touch the tray 7.

In the event that the tray 7 loaded on the rack 25 in the steam sterilization chamber 24 is too high or too low for the clamp mechanism 14, the user can adjust the height of the hanger member 8 having the clamp mechanism 14 by rotating the handle 11, to a height at which the tray 7 can be easily retrieved.

Once the tray 7 is clamped with the hanger member 8, the user rolls the tray transporting device 1 on its wheels 2 away from the high-temperature high-pressure steam sterilization device 21, so the hot tray 7 subjected to high-temperature high-pressure steam sterilization can be easily extracted.

Further, with the present embodiment, the user can place the tray 7, extracted while still hot, into the holding rack unit 22 having cooling functions, so that the scope 6 can be cooled and the user can use the scope 6 again for endoscope examination in a short time. With the tray clamped by the hanger member 8, the user rolls the tray transporting device 1 on its wheels 2 to the holding rack unit 22 by pushing the base 3 or the like of the tray transporting device 1, and places the scope 6, still in the tray 7, in the holding rack unit 22.

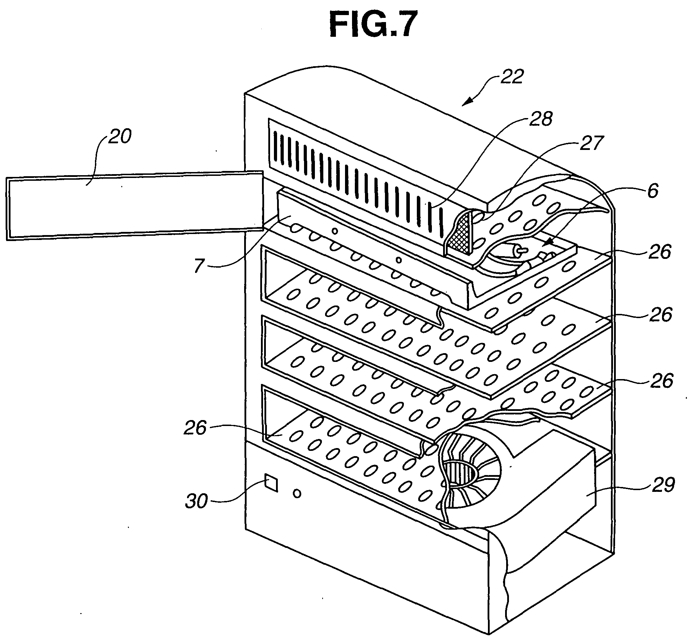

As shown in Fig. 7, upon opening the door 25, the holding rack unit 22 having cooling functions has holding racks 26 where the trays 7 can be loaded. The user moves the tray 7 onto the holding rack 26, releases the clamp mechanism 14, and thus releases the tray 7 from the hanger member 8.

The holding rack unit 22 having cooling functions is provided with an air duct 28 with a dust-removing filter 27 on the inner side of the top thereof, and a sirocco fan 29 serving as large-size air blowing means is provided below. The sirocco fan 29 blows air upwards, through the holding racks 26 disposed above, each of which has a great number of holes formed therein to facilitate passage of air.

After placing the tray 7 therein, the user closes the door 25 of the storage racks 26, and presses a switch 30, which blows air on the trays 7 and the like on the storage racks 26, so as to cool the scopes 6 while still in their trays 7. Upon passage of a predetermined amount of time, the user opens the door 25 of the storage racks 26 and removes the tray 7. The tray 7 and the scope 6 are cool enough to be handled with bare hands.

Thus, with the tray transporting device 1 of the steam sterilization system 10, trays 7 storing hot scopes 6 subjected to high-temperature high-pressure steam sterilization can be easily removed without directly touching the trays 7, and placed in the holding rack unit 22 having cooling functions, according to the present embodiment. Storing the trays 7 holding the hot scopes 6 subjected to high-temperature high-pressure steam sterilization in the holding rack unit 22 having cooling functions easily and quickly cools the scopes 6 to a state usable for endoscope examination.

Now, while the above description has been made with reference to an arrangement wherein hot scopes 6 in trays 7 are easily removed from the steam sterilization chamber 24 using the tray transporting device 1, the tray transporting device 1 may be used to directly clamp the sterilized scopes 6. In this case, the hanger member 8 and clamp mechanism should be formed so as to clamp the operating unit or the like of the scope 6, for example. Also, the length of the stay member 4 (and the height at which the slide unit 5 can be fixed) should be greater than at least the length of the insertion portion and universal cable of the scope 6, so that the tip of the insertion portion and the end of the universal cable do not come into contact with the floor in the event that the operating unit is clamped.

Incidentally, in the event that the user clamps directly and removes sterilized equipment other than scopes 6, the hanger member and clamp mechanism may be formed so as to readily clamp and release such sterilized equipment. In the tray transporting device 1 as shown in Fig. 5, the user can exchange the hanger member 8 provided with the clamp mechanism 14 by loosening the fixing screw 16. The hanger member provided with unshown clamp mechanism may be formed so that hot sterilized equipment can be easily removed from the steam sterilization chamber 24. Namely, the hanger member side of the slide unit 5 may be formed exchangeably. Different sizes and shapes of the tray 7 and directly clamping sterilized equipment can be considered.

Also, the lidded holding units 65 shown in Fig. 3 may be stored in the holding rack unit 22 having cooling functions, so as to cool.

Also, the trays 7 holding scopes 6 sterilized by the high-temperature.high-pressure steam sterilization device 21 shown in Fig. 4 may be stored in the cooling holding bins 34 in the storage unit 33 shown in Fig. 1A.

Moreover, the steam sterilization system 10 comprising the tray transporting device 1, high-temperature high-pressure steam sterilization device 21, and holding rack unit 22, shown in Fig. 4, may be configured using the storage unit 33 shown in Fig. 1A instead of the holding rack unit 22 having cooling functions.

As described above, modifications formed of any combination of parts or the like in the above-described embodiments belong to the present invention.

### Industrial Applicability

As described above, in a case of sterilizing the device such as the scope or the like with high-temperature steam, the steam sterilization storing device according to the present invention efficiently cools the device at a high-temperature following sterilization, thereby restoring the device to be usable again in a short time.

## Claims

1. A steam sterilization storing device comprising:
cooling holding means for holding and cooling sterilized equipment which has been sterilized by hot steam; and
at least one of
drying holding means for holding and drying the sterilized equipment, and
storage means for holding and storing the sterilized equipment.

2. A steam sterilization storing device according to Claim 1, wherein the cooling holding means is capable of holding containers storing the sterilized equipment.

3. A steam sterilization storing device according to Claim 1, wherein the drying holding means is capable of holding containers storing the sterilized equipment.

4. A steam sterilization storing device according to Claim 1, wherein the storage means is capable of holding containers storing the sterilized equipment.

5. A steam sterilization storing device according to Claim 1, wherein the cooling holding means, drying holding means, and storage means, are formed in a common unit.

6. A steam sterilization storing device according to Claim 1, wherein the cooling holding means comprises loading racks for loading the sterilized equipment.

7. A steam sterilization storing device according to Claim 1, wherein the cooling holding means has positioning portions for generally positioning the sterilized equipment to be held therein.

8. A steam sterilization storing device according to Claim 7, wherein the loading racks have positioning portions for generally positioning the sterilized equipment to be held thereby.

9. A steam sterilization storing device according to Claim 7, wherein the positioning portions are endoscope positioning portions for generally positioning endoscopes.

10. A steam sterilization storing device according to Claim 8, wherein the positioning portions are lowered portions corresponding to the shape of the sterilized equipment to be held thereby.

11. A steam sterilization storing device according to Claim 1, wherein the cooling holding means comprises fans for cooling the sterilized equipment to be held therein.

12. A steam sterilization storing device according to Claim 1, wherein the cooling holding means comprises pairs of fans for cooling the sterilized equipment to be held therein from above and below.

13. A steam sterilization storing device according to Claim 11, further comprising loading racks for loading the sterilized equipment, wherein portions of the loading racks facing the fans are formed of a mesh to facilitate passage of air.

14. A steam sterilization storing device according to Claim 1, wherein the cooling holding means is capable of being opened and closed by means of a door.

15. A steam sterilization storing device according to Claim 1, wherein the drying holding means is capable of being opened and closed by means of a door.

16. A steam sterilization storing device according to Claim 1, wherein the storage means is capable of being opened and closed by means of a door.

17. A steam sterilization storing device according to Claim 1, wherein the storage means is sectioned into storage areas according to the size of the sterilized equipment to be stored therein.

18. A steam sterilization storing device according to Claim 1, wherein containers for storing the sterilized equipment comprise detachable cooling members having cooling means.

19. A steam sterilization storing device according to Claim 18, wherein the cooling members are lids for covering the containers.

20. A steam sterilization storing device according to Claim 18, wherein the containers comprise positioning members for generally positioning the sterilized equipment.

21. A steam sterilization storing device comprising:
cooling holding means for holding and cooling sterilized equipment which has been sterilized by hot steam;
drying holding means for holding and drying the sterilized equipment; and
storage means for holding and storing the sterilized equipment.

22. A steam sterilization storing device according to Claim 21, wherein the cooling holding means is capable of holding containers storing the sterilized equipment.

23. A steam sterilization storing device according to Claim 21, wherein the drying holding means is capable of holding containers storing the sterilized equipment.

24. A steam sterilization storing device according to Claim 21, wherein the storage means is capable of holding containers storing the sterilized equipment.

25. A steam sterilization storing device according to Claim 21, wherein the cooling holding means, drying holding means, and storage means, are formed in a common unit.

26. A steam sterilization storing device according to Claim 21, wherein the cooling holding means comprises loading racks for loading the sterilized equipment.

27. A steam sterilization system comprising:
a high-temperature high-pressure steam sterilization device for storing and sterilizing equipment to be sterilized with hot steam;
a transporting device for holding and transporting the sterilized equipment sterilized in the high-temperature high-pressure steam sterilization device; and
a cooling holding device for holding and cooling sterilized equipment which has been sterilized by the high-temperature high-pressure steam sterilization device.

28. A steam sterilization system according to Claim 27, wherein the transporting device is capable of holding and transporting containers storing the sterilized equipment.

29. A steam sterilization system according to Claim 27, further comprising a drying holding device for holding and drying the sterilized equipment.

30. A steam sterilization system according to Claim 27, further comprising a storage device for holding and storing the sterilized equipment.

31. A steam sterilization system according to Claim 27, wherein the transporting device comprises
a hanger member for holding the sterilized equipment; and
a clamp mechanism capable of securing the sterilized equipment against the hanger member by clamping the sterilized equipment in operation with the hanger member to hold the sterilized equipment with the hanger member.

32. A steam sterilization system according to Claim 28, wherein the transporting device comprises
a hanger member for holding the container; and
a clamp mechanism capable of securing the container against the hanger member by clamping the sterilized equipment in operation with the hanger member to hold the container with the hanger member.

33. A steam sterilization system according to Claim 27, further comprising containers for storing the sterilized equipment, wherein the containers comprise detachable cooling members having air-cooled fans.

34. A steam sterilization storing device comprising:
a container storing sterilized equipment following sterilization with hot steam; and
a lid member, having small fans disposed at predetermined positions, which is attachable to the container.
